Europäisches Patentamt

European Patent Office

Office européen des brevets

(11) Veröffentlichungsnummer: **0 037 931**
A2

(12) # EUROPÄISCHE PATENTANMELDUNG

(21) Anmeldenummer: 81102213.6

(22) Anmeldetag: 24.03.81

(51) Int. Cl.³: **C 12 N 7/06,** A 61 K 39/145,
A 61 K 39/155, A 61 K 39/205

(30) Priorität: 14.04.80 DE 3014189

(43) Veröffentlichungstag der Anmeldung: 21.10.81
Patentblatt 81/42

(84) Benannte Vertragsstaaten: BE CH DE FR GB LI NL SE

(71) Anmelder: EUROPÄISCHES LABORATORIUM FÜR
MOLEKULARBIOLOGIE (EMBL), Meyerhofstrasse 1,
D-6900 Heidelberg 1 (DE)

(72) Erfinder: Simons, Kai, Prof. Dr., Otto-Hahn-Platz 7,
D-6900 Heidelberg-Emmertsgrund (DE)
Erfinder: Helenius, Ari, Dr., Otto-Hahn-Platz 7,
D-6900 Heidelberg-Emmertsgrund (DE)

(74) Vertreter: Weickmann, Heinrich, Dipl.-Ing Patentanwälte
Dipl.Ing.H.Weickmann et al, Dipl.Phys.Dr.K.Fincke
Dipl.Ing.F.A.Weickmann Dipl.Chem.B.Huber,
Dr.-Ing.H.Liska Möhlstrasse 22, D-8000 München 86 (DE)

(54) Verfahren zur Herstellung eines immunogenen Membranprotein-aggregats von Influenza-, Parainfluenza- und Rhabdo-Viren.

(57) Zur Herstellung von immunogen wirksamen lipid- und detergensfreien löslichen Aggregaten von amphiphilischen Membranproteinen von Parainfluenza-, Influenza- und Rhabdo-Viren mischt man den Virus mit einem nicht-ionischen oder Gallensäuren-Detergens, schichtet die Mischung in gepufferter wäßriger Salzlösung über einen detergensfreien Zuckergradienten, welcher seinerseits mit einer detergenshaltigen Zuckerlösung überschichtet ist, zentrifugiert bei mindestens 150 000 g, isoliert die proteinhaltige Fraktion, dialysiert gegen Pufferlösung und lyophilisiert die erhaltene Proteinmizellenlösung.

EP 0 037 931 A2

- 1 -

Verfahren zur Herstellung eines immunogenen Membranproteinaggregats von Influenza-, Parainfluenza- und Rhabdo-
Viren

Die Erfindung betrifft ein Verfahren zur Herstellung von
immunogen wirksamen, lipid- und detergensfreien löslichen Aggregaten von amphiphilischen Membranproteinen von
Influenza-, Parainfluenza- und Rhabdo-Viren.

Die Impfstoffe bzw. Vakzine, die gegenüber vielen, bei
Menschen und Tieren Krankheiten verursachenden, umhüllten Viren verfügbar sind, sind nicht nur ungenügend
wirksam, sondern sind ebenfalls nicht frei von unerwünschten Nebenwirkungen. Die Entwicklung auf dem Impfstoffgebiet erfolgt hauptsächlich in zwei Richtungen.
Einmal versucht man, lebende, abgeschwächte Viren als
Impfstoffe zu verwenden, und zum anderen versucht man,
verbesserte Impfstoffe auf Basis von Virusuntereinheiten (subunit vaccines) zu entwickeln. Bei den abgeschwächten lebenden Viren tritt die Schwierigkeit auf,
daß Stämme gefunden werden müssen, die gleichzeitig sicher und wirksam sind. Ernste Nebenwirkungen können sich
nur langsam entwickeln und sind schwer festzustellen.
Die Oberflächen-Glykoproteine umhüllter Viren werden als
Hauptantigene in den Virenteilchen angesehen, die eine schützende Immunantwort gegen Infektion induzieren. Ein wirksames Untereinheitsvakzin gegen ein solches Virus sollte daher eine Zubereitung aus den Antigenen der Virusoberfläche sein, von denen die anderen Komponenten des Virus abgetrennt sind. Versuche mit solchen Impfstoffen haben jedoch nur zweifelhafte Ergebnisse gezeigt. Beispielsweise wurde eine Reihe von Untereinheits-Vakzinen aus Influenzavirus hergestellt, aber diese sind bei der Verhinderung von Krankheiten bestenfalls nur teilweise wirksam, und sie waren nicht in der Lage, das Ausbreiten von Influenza-Epidemien einzudämmen. Eine Hauptschwierigkeit ist, daß die Virenoberflächen-Antigene integrale Membranproteine sind. Bevor sie von den anderen Virenproteinen und dem Virengenom getrennt werden können, müssen die Proteine solubilisiert werden. Das am meisten verwendete Verfahren zur Solubilisierung solcher Proteine besteht darin, daß man Detergentien verwendet. Diese binden jedoch die Proteine und können ihre antigene Potenz verändern. Wenn die Detergenskonzentration erniedrigt wird, kann das Protein ausfallen oder an verfügbaren Oberflächen durch hydrophobe Zwischenwirkungen haften. Die Wirksamkeit solcher durch Detergentien solubilisierten Virenoberflächen-Antigene als Impfstoff ist daher sehr schwierig vorherzusagen.

Aus der DE-OS 28 29 029 ist ein Verfahren der oben beschriebenen Art zur Herstellung von Untereinheit-Vakzinen von Parainfluenzavirus bekannt, bei welchem man die immunogen wirksame Glykoproteinkomponente mit einem kationischen Detergens selektiv extrahiert. Hierdurch gelingt es, durch andere Viruskomponenten, insbesondere das Genom, bedingte unerwünschte Nebenwirkungen und Komplikationen zu vermeiden. Die immunogene Wirksamkeit der so erhaltenen Untereinheits-Vakzine läßt jedoch zu wünschen übrig.

Der Erfindung liegt daher die Aufgabe zugrunde, Untereinheits-Vakzine gegen Influenza-, Parainfluenza- oder Rhabdo-Viren bedingte Erkrankungen zu schaffen, welche zwar die durch andere Virusbestandteile hervorgerufenen unerwünschten Nebenwirkungen nicht hervorrufen, andererseits jedoch eine sehr hohe immunogene Wirksamkeit aufweisen.

Gelöst wird diese Aufgabe erfindungsgemäß durch ein Verfahren zur Herstellung von immunogen wirksamen lipid- und detergensfreien löslichen Aggregaten von amphiphilischen Membranproteinen von Parainfluenza-, Influenza- und Rhabdo-Viren durch Extraktion der Membran mit oberflächenaktiven Mitteln, welches dadurch gekennzeichnet ist, daß man den Virus mit einem nicht-ionischen oder Gallensäuren-Detergens mischt, die Mischung in gepufferter wäßriger Salzlösung über einen detergensfreien Zukkergradienten schichtet, welcher seinerseits mit einer detergenshaltigen Zuckerlösung überschichtet ist, und bei mindestens 15o.ooo g zentrifugiert, die proteinhaltige Fraktion isoliert, gegen Pufferlösung dialysiert und die erhaltene Proteinmizellenlösung lyophilisiert.

Die erfindungsgemäß erhältlichen Proteinmizellen bestehen aus Aggregaten der immunogen wirksamen Spikeproteine mit einer rosettenartigen Struktur. Die Rosetten sind im Elektronenmikroskop sichtbar. Ihre sehr hohe immunogene Wirksamkeit wird darauf zurückgeführt, daß in diesen Rosetten die hydrophoben Enden der Spikeproteine sich zusammenlagern und die hydrophilen und immunogenen Enden nach außen gerichtet vorliegen und eine Konzentration an immunogenen Stellen aufweisen, die noch über der des intakten Virus liegt.

Zur Durchführung des Verfahrens der Erfindung wird zuerst der Virus in üblicher Weise aus einem geeigneten Gewebe gewonnen, konzentriert, beispielsweise durch Hohlfaserdialyse, und isoliert, beispielsweise durch Gradientenzentrifugation. Der gereinigte Virus wird dann mit dem nicht-ionischen Detergens oder dem Detergens auf Basis einer Gallensäure, welches im Überschuß angewendet wird, gemischt. Typische Beispiele für geeignete, nicht-ionische Detergentien sind die Polyglykolester und -äther mit aliphatischen und araliphatischen Säuren und Alkoholen. Besonders bevorzugt wird Triton X1oo (Octylphenoläther von Polyäthylenoxid). Typische Beispiele für geeignete Gallensäure-Detergentien sind Desoxycholat und Cholat.

Das Detergens wird, wie oben erwähnt, im Überschuß gegenüber der Virusmenge angewendet. Zweckmäßig mischt man Virus und Detergens im Gewichtsverhältnis 1 : 3 bis 1 : 1o.

Virus und Detergens werden in gepufferter Salzlösung zusammengegeben. Die Molarität der Salzlösung liegt zwischen o,o2 und o,5 M, vorzugsweise zwischen o,o5 und o,25 M. Besonders bevorzugt sind o,1 bis o,2 M. Als Salz wird Kochsalz bevorzugt, jedoch kommen auch andere Salze in Betracht, insbesondere mit Alkali- (einschließlich Ammonium) und Erdalkaliionen sowie den starken Mineralsäuren und organischen Säuren, wie Essigsäure, Trichloressigsäure, Ameisensäure und Oxalsäure.

Als Puffer eignen sich alle im angegebenen pH-Wertbereich puffernden Substanzen. Besonders gute Ergebnisse werden mit Tris-Puffer erhalten.

Die, wie oben beschrieben, hergestellte Virus/Detergens-Mischung wird auf einen Zuckergradienten, über dem eine Schicht einer detergenshaltigen Lösung des gleichen Zuckers liegt, geschichtet und bei mindestens 150.000 g zentrifugiert. Der Zuckergradient ist detergensfrei. Als Zucker wird für den Gradienten Saccharose bevorzugt, jedoch können auch andere Disaccharide und Monosaccharide sowie Glycerin, aber auch Tetrosen und Pentosen, verwendet werden.

Die Zuckerkonzentration im Gradienten weist zweckmäßig einen Wert von 15 bis 25 % als Anfangskonzentration, von 45 bis 60 % als Endkonzentration auf. Der Gradient kann beispielsweise aus einer oberen Schicht mit 15 bis 25 % Zuckergehalt und einer unteren Schicht mit 45 bis 60 % Zuckergehalt bestehen. Es können jedoch auch mehrere Schichten vorliegen, bei welchen die Konzentrationsdifferenzen zwischen den einzelnen Schichten dann entsprechend geringer sind.

Der Zuckergradient trägt eine Schicht aus einer Lösung des Zuckers, welche Detergens enthält. Die Konzentration des Detergens, welches das gleiche wie in der Virus/Detergens-Mischung ist, liegt zweckmäßig zwischen 0,25 und 3 %, vorzugsweise zwischen 0,75 und 1,5 %. Die Zuckerkonzentration kann gleich oder niedriger als in der Oberschicht des Gradienten sein.

Das erfindungsgemäß erhaltene Vakzin aus Spikeprotein-Aggregaten von Influenza- bzw. Parainfluenza-Viren wird zur Verabreichung zweckmäßig in einem geeigneten Lösungsmittel, wie z. B. physiologischer Kochsalzlösung, aufgenommen. Bevorzugt wird 0,1 M NaCl-Lösung, pH 7,2 bis 7,6. Der pH-Wert wurde mit 0,05 M Tris-HCl eingestellt, es können jedoch auch andere Puffer verwendet werden. Der so erhaltenen Lösung können auch übliche Adjuvantien zur Verstärkung der Wirksamkeit zugesetzt werden. Unter den Adjuvantien wird Freund's unvollständiges Adjuvans (besteht aus 2o % Falba in Bajol F) bevorzugt. Durch die Adjuvanszugabe kann die für die Immunantwort erforderliche Menge an Influenzamizellen herabgesetzt werden.

Zur weiteren Erläuterung dient die beigefügte Zeichnung. In dieser stellen dar:

Fig. 1 eine Darstellung der erfindungsgemäßen Schichtanordnung im Zentrifugengefäß bei Beginn der Zentrifugierung für Beispiel 1,

Fig. 2 ein SDS-Gelelektropherogramm der Aggregate (Mizellen) von Beispiel 1,

Fig. 3 ein SDS-Gelelektropherogramm der Aggregate von Beispiel 3.

Die Wirksamkeit eines erfindungsgemäßen Vakzins aus Parainfluenza-3-Virus (P 13-Virus) wurde an speziellen pathogenfreien (SPF) Schafen untersucht. Es ist bekannt, daß es möglich ist, SPF-Lämmer gegen P 13-Virus durch Vakzinierung zu schützen. Jedoch erwiesen sich Vakzine auf Basis von Ganzviren alleine als unwirksam (Res. Vet. Sc. 19, 56 (1975)). Bei Adjuvanszusatz war jedoch ein Schutz erzielbar. Das erfindungsgemäße Vakzin wurde nun in SPF-Lämmern untersucht, und zwar sowohl mit als auch ohne Adjuvanszusatz. Die Vergleichsgruppe erhielt kein Vakzin.

Die Einzelheiten der durchgeführten Versuche sind in den nachstehenden Tabellen 1 und 2 angegeben.

TABELLE 1

Behandlung

| Gruppe | Zahl der Tiere | Wochen nach Impfung | | | |
|---|---|---|---|---|---|
| | | 0 | 3 | 6 | 7 |
| A | 4 | nicht ge-impft | nicht ge-impft | mit P13-Virus I/N und I/T in-fiziert ($G_9$2/625/6) $10^7$ pfu) | Lämmer tö-ten und Autopsie |
| B | 7 | 15 μg Mi-zellen, TN-Puffer i.m. | 15 μg Mi-zellen,TN-Puffer,i.m. | " | " |
| C | 6 | 15 μg Mi-zellen, Ad-juvans: Ba-yol/Falba i.m. | 15 μg Mi-zellen, TN/Puffer i.m. | " | " |

Der empfindlichste Test auf Immunität ist die Virusabscheidung aus der Nase. Die hier erhaltenen Ergebnisse zeigt Tabelle 2.

TABELLE 2

Gewinnung von P 13-Virus aus nasalem Abstrich

| Gruppe | Tage nach der Infektion | | | | | | | Lunge |
|---|---|---|---|---|---|---|---|---|
| | 1 | 2 | 3 | 4 | 5 | 6 | 7 | |
| A | 4/4 | 3/4 | 4/4 | 4/4 | 4/4 | 4/4 | 0/4 | 0/4 |
| B | 7/7 | 6/7 | 7/7 | 7/7 | 5/7 | 0/7 | 0/7 | 0/7 |
| C | 1/6 | 0/6 | 1/6 | 1/6 | 1/6 | 0/6 | 0/6 | 0/6 |

Die obigen Ergebnisse zeigen, daß Gruppe C vollständig geschützt war. Dies wird durch die Autopsieergebnisse gestützt.Keinerlei Anzeichen von Pneumonie waren in dieser Gruppe erkennbar. Bei Gruppe B, welche zweimal 15 μg

0037931

Mizellen Protein ohne Adjuvans verabreicht erhalten hatte, wiesen die Tiere einen gewissen Schutz auf. Die Virusausscheidung hörte einen Tag eher auf als bei den Kontrollen und die Lungenläsionen waren deutlich geringer.

Die Versuchsergebnisse zeigen, daß mit der äußerst geringen Dosis von 15 µg Protein ein Schutz gegen ganz massive Virusinfektion ($10^9$ pfu!) erzielbar ist. Demgegenüber waren beispielsweise bei dem Vakzin gemäß DE-OS 28 29 089 zur Erzielung einer Immunantwort in Mäusen eine Injektion von 0,1 bis 1 mg Protein erforderlich und zur Erzielung von Immunität war eine zusätzliche Pusterinjektion notwendig. Vergleicht man das unterschiedliche Gewicht der Versuchstiere, so zeigt sich, daß das erfindungsgemäße Vakzin um wenigstens zwei bis drei Zehnerpotenzen wirksamer ist.

Die folgenden Beispiele erläutern die Erfindung weiter.

B e i s p i e l      1

Parainfluenza 3 (P 13)-Virus (ovine strain Moredun Institute Edinburgh, GB) wurde aus dem extrazellulären Medium von primären Nierenzellkulturen durch Konzentrieren mittels Hohlfaserdialyse und Isolierung durch Dichtegradientenzentrifugation gereinigt. Der Virus wurde als tablettenartiger Niederschlag in der Ultrazentrifuge gewonnen und in 0,1 M NaCl und 0,05 M Tris, pH 7,4, aufgenommen. Die Proteinkonzentration betrug 5 mg/ml.

1 mg Virus wurde mit 5 mg Triton X100 versetzt. Die erhaltene Lösung wurde über einen Saccharosegradienten geschichtet. Der Saccharosegradient bestand aus einer Saccharoselösung mit von 20 bis 50 % linear ansteigendem

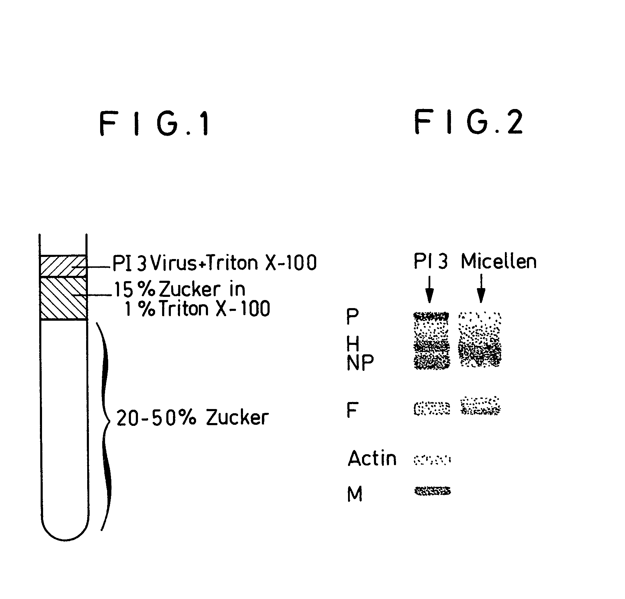

Saccharosegehalt im gleichen Tris-NaCl-Puffer, wie oben beschrieben. Über dem Saccharosegradienten befand sich eine Schicht einer 15 %-igen Saccharoselösung mit 1 % Triton X1oo im gleichen Puffer. Dann wurde 22 Stunden bei 4oooo UPM auf einer SW4oSpinko-Zentrifuge (ca. 19o.ooo g) zentrifugiert. Fig. 1 der Zeichnung zeigt die Anordnung der Schichten im Zentrifugenglas. Nach Ender der Zentrifugierung befand sich das Nucleokapsid in dem Niederschlag und das Spikeprotein in der Mitte des Gradienten. Die spikeproteinhaltigen Fraktionen wurden vereinigt, gegen 1/1o TN-Puffer über Nacht mit einmaligem Austausch des Puffers dialysiert und dann lyophilisiert.

Die Untersuchung des Lyophilisats im Elektronenmikroskop ergab rosettenähnliche Strukturen. Der S-Wert der Proteinmizellen war 32 S. Das Ergebnis der SDS-Gelelektrophorse zeigt Fig. 2.

B e i s p i e l   2

In analoger Weise, wie oben beschrieben, wurden Viren der Myxogruppe (Hühnerfleckeninfluenza-Fowlplaque) als Ausgangsmaterial für die Vakzingewinnung eingesetzt unter Bildung detergensfreier Spikeproteinaggregate. Die Dauer der Zentrifugierung betrug 19 Stunden. Das Virus wurde vom Institut für Virologie, Universität Gießen, erhalten.

B e i s p i e l   3

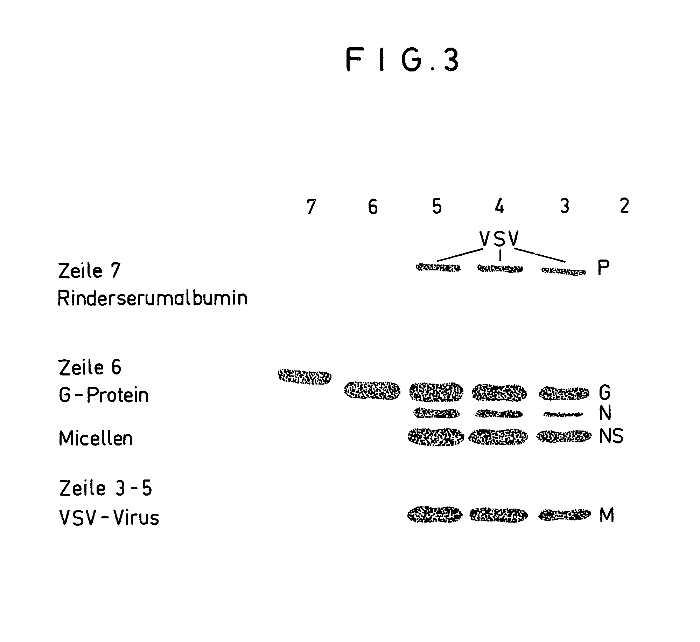

Wie in Beispiel 1 beschrieben, wurde Vakzin des Rhabdo- Virus VSV (vesikulärer Stomatitis-Virus, Animal Virus Research Institute, Pirbright, Woking Surrey, GB) hergestellt. Als Puffer wurde jedoch o,o5 M Tris, pH 8, enthaltend o,2 M NaCl, verwendet. Zentrifugiert wurde
24 Stunden. Fig. 3 zeigt das Ergebnis der SDS-Gelelektrophorese der erhaltenen G-Protein-Mizellen.

Patentansprüche

1. Verfahren zur Herstellung von immunogen wirksamen lipid- und detergensfreien löslichen Aggregaten von amphiphilischen Membranproteinen von Parainfluenza-, Influenza- und Rhabdo-Viren durch Extraktion der Membran mit oberflächenaktiven Mitteln, dadurch g e k e n n - z e i c h n e t , daß man den Virus mit einem nicht-ionischen oder Gallensäuren-Detergens mischt, die Mischung in gepufferter wäßriger Salzlösung über einen detergensfreien Zuckergradienten schichtet, welcher seinerseits mit einer detergenshaltigen Zuckerlösung überschichtet ist, und bei mindestens 150.000 g zentrifugiert, die proteinhaltige Fraktion isoliert, gegen Pufferlösung dialysiert und die erhaltene Proteinmizellenlösung lyophilisiert.

2. Verfahren nach Anspruch 1, dadurch g e k e n n - z e i c h n e t , daß man als Zucker Saccharose verwendet.

3. Verfahren nach Anspruch 1 oder 2, dadurch g e - k e n n z e i c h n e t , daß man beim Zuckergradienten eine Anfangskonzentration von 15 bis 25 % und eine Endkonzentration von 45 bis 6o % einsetzt.

4. Verfahren nach einem der vorhergehenden Ansprüche, dadurch g e k e n n z e i c h n e t , daß man Virus und Detergens im Gewichtsverhältnis 1 : 3 bis 1 : 1o mischt.

5. Verfahren nach einem der vorhergehenden Ansprüche, dadurch g e k e n n z e i c h n e t , daß man eine o,o5 bis o,2 molare NaCl-Lösung verwendet.

0037931

6. Verfahren nach einem der vorhergehenden Ansprüche, dadurch g e k e n n z e i c h n e t , daß man einen pH-Wert zwischen 6,5 und 8,o einstellt.

7. Verfahren nach einem der vorhergehenden Ansprüche, dadurch g e k e n n z e i c h n e t , daß man die Proteinmizellen in Freund's unvollständigem Adjuvans oder einem anderen Adjuvans auflöst.

# F I G.1

PI 3 Virus+Triton X-100

15% Zucker in
1% Triton X-100

20-50% Zucker

# F I G.2

PI 3  Micellen

P
H
NP

F

Actin

M

# FIG.3

Zeile 7
Rinderserumalbumin

Zeile 6
G-Protein

Micellen

Zeile 3-5
VSV-Virus